# FASCICULE DE BREVET EUROPEEN

(11) **EP 0 988 271 B1**
(45) Date de publication et mention de la délivrance du brevet: **20.08.2003**
(21) Numéro de dépôt: 98929491.3
(22) Date de dépôt: 04.06.1998
(51) Int. Cl.: C07C 49/92, C08L 27/06

(54) **ACETYLACETONATE DE CALCIUM OU DE MAGNESIUM, ENROBE ET SON UTILISATION COMME STABILISANT DE POLYMERES HALOGENES**
BESCHICHTETES CALCIUM ODER MAGNESIUM ACETYLACETONAT, UND IHRE VERWENDUNG ALS STABILISATOR FÜR HALOGENIERTE POLYMERE
COATED CALCIUM OR MAGNESIUM ACETYLACETONATE, AND ITS USE FOR STABILISING HALOGENATED POLYMERS

(30) Priorité: 04.06.1997 FR 9706859
(43) Date de publication de la demande: 29.03.2000
(73) Titulaire: RHODIA CHIMIE, 92408 Courbevoie Cédex (FR)
(72) Inventeur: GAY, Michel, F-69100 Villeurbanne (FR); HENRIO, Françoise, F-78630 Morainvilliers (FR)
(74) Mandataire: Wattremez, Catherine
(86) Numéro de dépôt international: FR9801140
(87) Numéro de publication internationale: WO98055440

(56) Documents cités:
- EP-A- 0 336 289
- EP-A- 0 564 035
- DE-A- 19 610 320
- DATABASE WPI Section Ch, Week 9314 Derwent Publications Ltd., London, GB; Class A18, AN 93-112918 XP002053053 & JP 05 051565 A (AICA KOGYO CO LTD) , 2 mars 1993

## Description

La présente invention a pour objet des particules comprenant de l'acétylacétonate de calcium ou de magnésium, au moins partiellement recouvert par un agent compatibilisant, ainsi que son obtention.

Elle a de même pour objet l'utilisation de ces particules comme additif dans des formulations à base de polymères halogénés.

L'acétylacétonate de calcium figure parmi les stabilisants thermiques les plus usuels pour les formulations comprenant des polymères halogénés, et plus spécialement du polychlorure de vinyle.

Cependant, s'il est clairement établi que les formulations de polymères halogénés peuvent être efficacement stabilisées vis-à-vis de la température, il n'en reste pas moins que la mise en oeuvre de polymères ainsi stabilisés présente quelques difficultés. En effet, on a constaté que la présence de ce chélate précis était la cause de défauts dans le polymère mis en forme. Plus particulièrement, on a remarqué que les articles obtenus pouvaient présenter des hétérogénéités ayant l'aspect de cratères, de grains ou encore de piqûres.

Ces mêmes difficultés sont attendues avec un acétylacétonate de magnésium.

Dans la demande de brevet EP 339289, qui a pour objet une composition stabilisante comprenant au moins un complexe de β-dicétone et de calcium, des esters d'acides carboxyliques et de polyols, des sels de zinc d'acides carboxyliques, il est recommandé de préparer l'acétylacétonate de calcium in situ afin d'améliorer la dispersion de l'acétylacétonate de calcium au sein de la composition stabilisante et donc de limiter l'apparition de défauts dans le polymère mis en forme.

L'un des buts de la présente invention est donc de proposer une solution aux problèmes des hétérogénéités apparaissant lors de la mise en forme de formulations à base de polymère(s) halogéné(s) et stabilisées par l'acétylacétonate de calcium ou de magnésium.

Ainsi, il a été trouvé de façon tout à fait inattendue que l'association d'acétylacétonate de calcium ou de magnésium, avec un composé particulier, recouvrant au moins en partie ledit chélate, permettait d'éliminer les problèmes d'hétérogénéités mentionnés auparavant dans la formulation polymérique. Le composé recouvrant au moins partiellement le chélate permet, de ce point de vue, de rendre l'acétylacétonate de calcium ou de magnésium, compatible avec la formulation.

Il est à noter que l'enrobage peut de même avoir un rôle d'hydrophobation de l'acétylacétonate de calcium ou de magnésium, diminuant, voire évitant, la reprise d'humidité par ces composés. De cette façon, les propriétés d'usage de la formulation polymérique finale, telles que notamment la résistance à la soudure, sont améliorées par voie de conséquence.

Enfin, l'enrobage permet d'améliorer la dispersion de l'acétylacétonate de calcium ou de magnésium, dans la formulation comprenant le polymère halogéné.

Ainsi, un premier objet de la présente invention est constitué par des particules comprenant de l'acétylacétonate de calcium ou de magnésium, recouvert partiellement ou en totalité par au moins un agent compatibilisant choisi parmi :
□ les alcools comprenant 12 à 30 atomes de carbone, saturés ou non ;
□ les acides carboxyliques ou sulfoniques, comprenant 12 à 30 atomes de carbone, saturés ou non, substitués ou non par au moins un groupement hydroxyle, ou leurs dérivés ;
□ les phosphates ou les titanates comprenant au moins une chaîne comprenant 12 à 30 atomes de carbone, saturée ou non ;
□ les composés β-dicétoniques présentant au moins une chaîne comprenant au moins 7 atomes de carbone ;
□ les cires ;
□ les polyols ;
□ les huiles végétales époxydées ;
□ les huiles ou les résines polysiloxaniques ou encore les silanes.

Un second objet de la présente invention est constitué par un procédé de préparation de l'additif précité, dans lequel on met en contact l'acétylacétonate de calcium ou de magnésium, avec au moins un agent compatibilisant, éventuellement mis sous la forme d'une suspension ou d'une dispersion.

Un autre but de la présente invention concerne l'utilisation d'un tel composé en tant qu'additif dans des formulations comprenant au moins un polymère halogéné.

Mais d'autres caractéristiques et avantages de la présente invention apparaîtront plus clairement à la lecture de la description qui va suivre.

L'acétylacétonate métallique entrant dans la présente invention correspond à la formule suivante [CH₃COCHCOCH₃]₂M, x.H₂O, dans laquelle x est compris entre 0 et 2, et M représente le calcium ou le magnésium. La présente invention est particulièrement appropriée pour l'acétylacétonate de calcium.

L'acétylacétonate de calcium est bien connu, et on le trouve par exemple dans le commerce sous la dénomination Rhodiastab X7®, commercialisé par Rhodia Chimie.

Dans ce qui va suivre, et pour des raisons de simplification de l'exposé, il ne sera fait référence qu'à l'acétylacétonate, sachant que ce terme se rapportera à la fois à l'acétylacétonate de calcium ou à l'acétylacétonate de magnésium.

On ne sortirait pas du cadre de la présente invention en utilisant une combinaison de ces deux acétylacétonates.

Généralement l'acétylacétonate est mis en oeuvre sous la forme d'une poudre dont la granulométrie est comprise entre 3 et 200 µm.

Selon une caractéristique essentielle de la présente invention, l'acétylacétonate est recouvert en partie ou totalement par au moins un agent compatibilisant.

Cet agent compatibilisant est plus particulièrement choisi parmi :
□ les alcools comprenant 12 à 30 atomes de carbone, saturés ou non ;
□ les acides carboxyliques ou sulfoniques, comprenant 12 à 30 atomes de carbone, saturés ou non, substitués ou non par au moins un groupement hydroxyle, ou leurs dérivés ;
□ les phosphates ou les titanates comprenant au moins une chaîne comprenant 12 à 30 atomes de carbone, saturée ou non ;
□ les composés β-dicétoniques présentant au moins une chaîne comprenant au moins 7 atomes de carbone ;
□ les cires ;
□ les polyols ;
□ les huiles végétales époxydées ;
□ les huiles ou les résines polysiloxaniques ou encore les silanes.

En ce qui concerne les alcools comprenant 12 à 30 atomes de carbone, conviennent particulièrement les monoalcools aliphatiques, saturés ou non. A titre d'exemple, on peut citer sans intention de s'y limiter les alcools laurique, myristique, stéarique, isostéarique, cétylique, behénique, lauroléique, oléique, érucique, linoléique, seuls ou en mélange.

Parmi les agents compatibilisants utilisables dans le cadre de la présente invention, figurent les acides carboxyliques comprenant 12 à 30 atomes de carbone, ainsi que leurs dérivés.

Plus particulièrement, on emploie en tant qu'agent compatibilisant, les acides carboxyliques aliphatiques, comprenant 12 à 30 atomes de carbone, saturés ou non, linéaires ou ramifiés, et comprenant éventuellement un ou plusieurs groupes hydroxyle.

En tant qu'agent de ce type, on peut mentionner, entre autres, les acides stéarique, laurique, myristique, palmitique, oléïque, ricinoléïque, béhénique (docosanoïque), l'acide linoléique, l'acide linolénique, l'acide ricinoléique, l'acide hydroxystéarique, ou tout autre acide provenant des glycérides ou triglycérides, naturels ou non, conviennent à la mise en oeuvre de l'invention. Les acides peuvent être mis en oeuvre seuls ou en mélange.

En ce qui concerne les dérivés possibles de ces acides, on peut citer les esters de tels acides. Conviennent notamment les esters obtenus à partir de monoalcools comprenant 1 à 30 atomes de carbone, ou les mono- ou polyesters obtenus à partir de polyols, comme par exemple des dérivés du glycérol, des alkylènes glycols comme le propylène glycol.

Les sels des acides carboxyliques précités sont une autre classe de dérivés de ces acides. Sont particulièrement convenables, les sels de métaux alcalins, alcalino-terreux, d'aluminium, de lanthane et de zinc. Plus particulièrement, on emploie des sels de sodium, de calcium, de magnésium, d'aluminium, de lanthane et de zinc.

A titre d'acides sulfoniques convenables, on peut citer l'acide dodécylbenzène sulfonique.

Parmi les agents compatibilisants utilisables dans le cadre de la présente invention, figurent les composés β-dicétoniques (ou β-dicétones) de formule R¹COCHR²COR³ ; dans laquelle le radical R¹, représente un radical hydrocarboné, linéaire ou ramifié, substitué ou non, en C₇-C₃₀, le radical R³ représente un radical hydrocarboné, linéaire ou ramifié, substitué ou non, en C₁-C₃₀, le radical R² représente un atome d'hydrogène ou un radical hydrocarboné, linéaire ou ramifié, en C₁-C₄.

Plus particulièrement, le radical R¹ représente un radical alkyle, alcényle, linéaire ou ramifié, en C₇-C₃₀ ; le radical et R³ représente un radical alkyle, alcényle, linéaire ou ramifié, en C₁-C₃₀ ; un radical aryle en C₆-C₃₀, substitué ou non par au moins un radical alkyle et/ou un atome d'halogène et/ou un atome de silicium ; un radical cycloaliphatique en C₃-C₁₄ et pouvant éventuellement comporter des liaisons doubles carbone - carbone. Il est à noter que les radicaux R¹ et R³ peuvent être identiques ou différents.

De préférence, le radical R³, représentent un radical alkyle, linéaire ou ramifié en C₁-C₁₈ ; un radical aryle en C₆-C₁₀, substitué ou non par au moins un radical alkyle et/ou un atome d'halogène ; ou un radical cycloaliphatique en C₃-C₁₄ et pouvant éventuellement comporter des liaisons doubles carbone - carbone.

Les radicaux R¹ et R³ qui viennent d'être décrits peuvent être éventuellement modifiés (substitués) par la présence dans la chaîne aliphatique d'un ou plusieurs groupes de formule -O-, -CO-O-, -CO-.

Le radical R² peut être soit un atome d'hydrogène, soit un radical alkyle en C₁-C₄, dont la chaîne aliphatique peut être interrompue (substitué) par un ou plusieurs groupes de formule -O-, -CO-O-, -CO-.

De préférence R² représente un atome d'hydrogène .

A titre de composé β-dicétonique convenable, on peut citer entre autres, l'octanoylbenzoylméthane, le stéaroylbenzoylméthane, le palmitoylbenzoylméthane, le lauroylbenzoylméthane.

Parmi les cires utilisables comme agent compatibilisant de l'acétylacétonate , on peut mentionner notamment les cires montanates, les cires de poyléthylène ou leur dérivés oxydés, ainsi que les paraffines.

Un autre type d'agent compatibilisant est constitué par les polyols, que les fonctions hydroxyles soient vicinaux (en position α ou β), ou non. Ces composés peuvent être utilisés seuls ou en mélange.

Plus particulièrement, on peut mettre en oeuvre des polyols comprenant 2 à 32 atomes de carbone, présentant deux à neuf groupements hydroxyles, et dont les groupement hydroxyles se trouvent indifféremment en position vicinale ou non.

Parmi ces composés on peut mentionner les diols en C₂-C₃₂ tels que le propylène glycol, butylèneglycol, le butanediol, pentanediol, l'hexanediol, le dodécanediol, le néopentylglycol, les polyols tels que le triméthylolpropane, le pentaérythritol, le dipentaérythritol, le tripentaérythritol, le xylitol, le mannitol, le sorbitol, la glycérine, les mélanges d'oligomères de la glycérine présentant un degré de polymérisation de 2 à 10, l'alcool hydroxystéarique, l'alcool ricinoléique.

Une autre famille de polyols pouvant être convenablement mise en oeuvre, est constituée par les alcools polyvinyliques éventuellement partiellement acétylés.

On peut de même utiliser des composés hydroxylés comprenant des groupements isocyanurates, tels que par exemple le tris (2-hydroxyéthyl) isocyanurate.

Selon une autre possibilité, l'agent compatibilisant est choisi parmi les huiles végétales époxydées telles que l'huile de soja époxydée, l'huile de ricin époxydée.

Un autre type d'agent compatibilisant peut être choisi parmi les huiles ou les résines polysiloxaniques.

Plus particulièrement, on peut citer les huiles polydialkylsiloxaniques ou les huiles polyhydrogénoalkylsiloxaniques, pour lesquelles le radical alkyle comprend 1 à 3 atomes de carbone, et de préférence correspond à un radical méthyle.

Ces huiles correspondent à la formule générale suivante :
YO-[(R)Si(R)-O]ₓ-Y, formule dans laquelle R, identiques ou différents, représentent un radical alkyle comprenant 1 à 3 atomes de carbone, et de préférence un méthyle, ou un atome d'hydrogène à la condition que seulement l'un des deux radicaux soit un hydrogène, Y représente un atome d'hydrogène ou (R')₃Si, avec R', identiques ou différents, représentant un radical alkyle comprenant 1 à 3 atomes de carbone, de préférence le méthyle. Le coefficient x varie dans un large domaine, mais plus particulièrement il est compris entre 5 et 300.

Conviennent aussi les huiles polyméthylsiloxanes fonctionnalisées comme par exemple les huiles γ-hydroxypropylénées.

Enfin, pour ce qui est des résines polysiloxaniques, on emploie tout particulièrement les résines obtenues par l'action d'huiles polyhydrogénosiloxaniques sur des huiles polysiloxaniques portant des groupement vinyles, en présence d'un catalyseur à base de platine.

On peut de même employer des résines polysiloxaniques obtenues par hydrolyse et auto-condensation d'au moins un silane de formule (RO)₃SiF, ou (RO)₂(Me)SiF, dans lesquelle R, identiques ou différents, représentent un radical alkyle comprenant 1 à 4 atomes de carbone, F représente plus particulièrement les radicaux suivants :
- CH=CH₂.
-(CH₂)₃OH,
-(CH₂)₃-NH₂,
-(CH₂)₃NHCH₂CH₂NH₂,

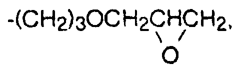

-(CH₂)₃O-CO-CH=CH₂,
-(CH₂)₃O-CO-(CH₃)CH=CH₂.

On peut utiliser aussi les silanes précités en tant qu'agents compatibilisants.

Selon la présente invention, l'agent compatibilisant peut être employé seul ou sous la forme d'un mélange quelconque entre plusieurs des agents possibles précités. Dans ce cas deux alternatives sont possibles, soit un revêtement des particules d'acétylacétonate comprenant plusieurs agents, soit un mélange de particules comprenant chacune un revêtement à base d'un seul agent.

La proportion en poids d'agent compatibilisant par rapport à l'acétylacétonate (c'est-à-dire l'acétylacétonate de calcium ou l'acétylacétonate de magnésium) peut varier dans un domaine aussi large que 0,1 à 20 % en poids par rapport au poids d'acétylacétonate. Plus particulièrement, la proportion en agent compatibilisant est comprise entre 0,1 et 10 % en poids par rapport au poids d'acétylacétonate. De préférence, la proportion varie entre 0,1 et 5 % en poids par rapport à la même référence, et de manière encore plus avantageuse entre 0,1 et 2 % en poids par rapport à la même référence.

Un procédé de préparation de l'additif selon l'invention va maintenant être décrit.

Les procédés classiques d'enrobage peuvent être employés pour préparer le composé selon l'invention.

Ainsi, le composé selon l'invention peut être obtenu en mettant en contact l'acétylacétonate de calcium ou de magnésium avec au moins un agent compatibilisant, éventuellement en présence d'un solvant et/ou d'un dispersant.

Il est à noter que le solvant et/ou le dispersant mis en oeuvre ne solubilisent pas l'acétylacétonate de calcium ou de magnésium.

Habituellement, le solvant ou dispersant de l'agent compatibilisant, s'il est présent, est choisi parmi l'eau, les monoalcools en C₁-C₅, notamment tels que le méthanol, l'éthanol, les éthers en C₂-C₆ comme le diméthyléther, le méthyléthyléther, le diéthyléther, les hydrocarbures comme entre autres l'hexane. Le mélange de ces solvants/dispersants est bien entendu possible.

Selon une première variante, que l'on pourrait qualifier d'imprégnation à sec, la quantité de solvant/dispersant est telle que l'on ne dépasse pas la limite de la capacité d'absorption de l'acétylacétonate de calcium ou de magnésium. L'homme du métier, en fonction des conditions de mise en oeuvre (quantité, granulométrie, surface spécifique de l'acétylacétonate, quantité d'agent compatibilisant) est tout à fait à même de déterminer la quantité optimale de solvant/dispersant.

Selon un autre mode de réalisation de cette variante, l'agent compatibilisant est mis en contact avec l'acétylacétonate, en l'absence de solvant ou de dispersant.

La mise en contact avec l'acétylacétonate, selon l'un ou l'autre mode de réalisation indiqué ci-dessus (présence ou absence de solvant/dispersant) a de préférence lieu de manière à contrôler, dans le but de l'éviter, toute agglomération des particules entre elles. Par conséquent, selon cette première variante, on préfère introduire l'agent comptabilisant dans l'acétylacétonate.

Cette mise en contact peut avoir lieu au moyen d'une burette ou de tout appareil d'introduction de ce type. Elle peut aussi avoir lieu au moyen d'un pulvérisateur muni d'une buse.

La mise en contact a bien évidemment lieu sous agitation, que celle-ci soit causée par l'emploi d'un agitation mécanique ou bien par l'emploi d'un tambour tournant, ou d'un granulateur.

La durée de l'opération dépend de toutes sortes de critères. Mais en général elle a lieu jusqu'à ce que l'on obtienne un mélange macroscopiquement homogène, en d'autres termes un mélange dépourvu d'agrégats visibles.

Selon une seconde variante, la quantité de solvant mise en oeuvre est telle qu'elle solubilise la quantité souhaitée d'agent compatibilisant. Là encore, l'homme du métier, avec ses seules connaissances générales peut déterminer cette quantité. Classiquement, par cette seconde variante, on obtient des dispersions d'acétylacétonate dans une solution d'agent(s) compatibilisant(s). Il est à noter qu'une fraction de l'agent compatibilisant peut toujours se trouver sous la forme d'une dispersion.

La mise en contact selon cette variante peut avoir lieu en introduisant indifféremment l'acétylacétonate dans la solution, ou le contraire, voire en mettant simultanément les deux en contact.

Cette mise en contact a lieu sous agitation mécanique, complétée si nécessaire par l'emploi d'ultrasons.

Quelle que soit la variante retenue, la mise en contact a lieu de préférence à température ambiante bien que des températures plus élevées ne soient pas exclues.

L'opération de mise en contact a lieu de manière avantageuse sous air.

En général, une fois l'acétylacétonate et l'agent compatibilisant mis en contact, on procède à un séchage. Le séchage peut avoir lieu à température ambiante, en étuve, ou bien par évaporation, sous vide ou non du solvant/dispersant s'il est présent. De préférence, et cela s'applique plutôt dans le cas de la seconde variante, on effectue le séchage de manière à éviter une perte trop rapide en solvant/dispersant, ce qui aurait pour effet d'agglomérer les particules entre elles.

La durée de l'opération de séchage est habituellement comprise entre quelques minutes à 12 heures environ.

Eventuellement, et si nécessaire, avant introduction dans la formulation polymérique, le produit enrobé peut subir un léger broyage afin de désagglomérer les particules.

Bien entendu, ce procédé n'est donné qu'à titre indicatif et toute autre méthode visant à enrober un produit par un autre peut être employée.

Ainsi que cela a été indiqué auparavant, l'additif selon l'invention est plus particulièrement destiné à être employé dans des formulations comprenant des polymères halogénés. Plus particulièrement les polymères en question sont des polymères chlorés.

L'invention est particulièrement bien appropriée à la stabilisation de formulations à base de polychlorure de vinyle (PVC).

Par polychlorure de vinyle, on entend des compositions dont le polymère est un homopolymère de chlorure de vinyle. L'homopolymère peut être modifié chimiquement par exemple par chloration.

De nombreux copolymères du chlorure de vinyle peuvent également être stabilisés en utilisant la composition selon l'invention. Ce sont en particulier des polymères obtenus par copolymérisation du chlorure de vinyle avec des monomères présentant une liaison éthyléniquement polymérisable, comme par exemple l'acétate de vinyle, le chlorure de vinylidène ; les acides maléique, fumarique ou leurs esters ; les oléfines telles que l'éthylène, le propylène, l'hexène ; les esters acryliques ou méthacryliques ; le styrène ; les éthers vinyliques tels que le vinyldodécyléther.

Habituellement les copolymères contiennent au moins 50 % en poids de motifs de chlorure de vinyle et de préférence au moins 80 % en poids de tels motifs.

Le PVC seul ou en mélange avec d'autres polymères est le polymère chloré le plus largement utilisé dans les formulations stabilisées selon l'invention.

D'une manière générale, tout type de polychlorure de vinyle convient, quel que soit son mode de préparation. Ainsi les polymères obtenus par exemple en mettant en oeuvre des procédés en masse, en suspension, en émulsion peuvent être stabilisés en utilisant la composition selon l'invention, et ceci quelle que soit la viscosité intrinsèque du polymère.

La présence de l'additif selon l'invention dans la formulation permet d'améliorer la compatibilité de l'acétylacétonate dans lesdites formulations polymériques, afin d'éviter les problèmes d'hétérogénéité causés par la présence de l'acétylacétonate seul, lors de la mise en oeuvre de la formulation. Elle permet de même d'augmenter la dispersion de l'acétylacétonate dans la formulation.

La quantité d'additif selon l'invention, exprimée en acétylacétonate de calcium ou de magnésium, est comprise entre 0,01 et 5 g pour 100 g de polymère halogéné, plus particulièrement, entre 0,05 et 2 g par rapport à la même référence.

Les formulations polymériques peuvent en outre comprendre les additifs usuels dans le domaine.

Ainsi, la formulation peut comprendre au moins un composé β-dicétonique, qui peut être sous forme libre, ou sous la forme d'un chélate métallique, ou encore sous la forme d'une combinaison de ces deux espèces.

Ainsi, lorsque le composé se trouve sous une forme libre, il correspond à la formule (I) suivante R¹COCHR²COR³ ; formule dans laquelle R¹ et R³, semblables ou différents, représentent chacun un radical hydrocarboné, linéaire ou ramifié, substitué ou non, en C₁-C₃₀ ; R² est un atome d'hydrogène ou un radical hydrocarboné, linéaire ou ramifié, en C₁-C₄.

Lorsque le composé β-dicétonique se trouve sous la forme d'un chélate métallique, il peut être représenté par la formule (II) suivante : 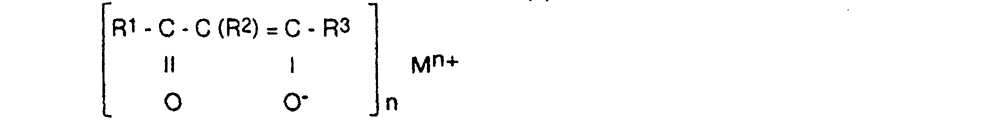 formule dans laquelle Mⁿ⁺ représente l'un au moins des métaux suivants :calcium, de zinc, d'aluminium, de magnésium ou de lanthane, n étant égal à 2 ou 3, R¹, R³, identiques ou différents, linéaires ou ramifiés, substitués ou non, représentent un radical hydrocarboné en C₁-C₃₀ et R² représente un atome d'hydrogène ou un radical hydrocarboné, linéaire ou ramifié, en C₁-C₄, à l'exception des acétylacétonates de calcium et de magnésium.

Selon un mode de réalisation plus particulier de l'invention, les radicaux R¹ et R³, identiques ou différents, représentent un radical alkyle, alcényle, linéaire ou ramifié, en C₁-C₂₄ ; un radical aryle en C₆-C₃₀, substitué ou non par au moins un radical alkyle et/ou un atome d'halogène et/ou un atome de silicium ; un radical cycloaliphatique en C₃-C₁₄ et pouvant éventuellement comporter des liaisons doubles carbone - carbone.

Il est à noter que si le composé β-dicétonique est présent sous les deux formes précitées, les radicaux R¹ et R³ peuvent être différents d'un produit à l'autre.

De préférence, les radicaux R¹ et R³, identiques ou différents, représentent un radical alkyle, linéaire ou ramifié en C₁-C₁₈ ; un radical aryle en C₆-C₁₀, substitué ou non par au moins un radical alkyle et/ou un atome d'halogène ; ou un radical cycloaliphatique en C₃-C₁₄ et pouvant éventuellement comporter des liaisons doubles carbone - carbone.

Selon une autre variante, les radicaux R¹ et R³ peuvent être reliés entre eux de manière à ce que le composé β-dicétonique se trouve sous la forme d'un cycle.

Les radicaux R¹ et R³ qui viennent d'être décrits peuvent être éventuellement modifiés (substitués) par la présence dans la chaîne aliphatique d'un ou plusieurs groupes de formule -O-, -CO-O-, -CO-.

Le radical R² peut être soit un atome d'hydrogène, soit un radical alkyle en C₁-C₄, dont la chaîne aliphatique peut être interrompue par un ou plusieurs groupes de formule -O-, -CO-O-, -CO-.

De préférence R² représente un atome d'hydrogène.

Les composés β-dicétoniques peuvent être obtenus selon les méthodes classiques.

Par exemple, les β-dicétones peuvent être synthétisées en mettant en oeuvre une réaction de condensation d'un ester sur une cétone, en présence d'un agent alcalin qui peut être un amidure d'un cation comme le sodium, ou de l'hydrogène.

Cette réaction a notamment été décrite dans les publication suivantes : *R*. *HAUSER et coll. "The acylation of ketones to form diketones", Organic Reactions - vol. VII, chapter 3, p. 59* - *196, John WILER, Ed. New York (1954), WIEDMAN et coll., C. R. 238 (1954), p. 699, MORGAN et coll., BER. 58 (1925), p. 333, LIVINGSTONE et Coll., Am. Soc. 46 (1924), p. 881-888, R. LEVINE et Coll. Am. Soc. 67 (1945)*, *p. 1510-1517,* le brevet européen EP 596 809.

A titre d'exemple de composés β-dicétoniques convenant à la mise en oeuvre de invention, on peut citer notamment sans intention de s'y limiter, l'octanoyl-benzoylméthane, le stéaroylbenzoylméthane, le dibenzoylméthane ou encore l'acétylbenzoylméthane, seuls ou en mélange. Il est à noter que l'on peut mettre en oeuvre les produits purifiés ou non.

Les produits commerciaux suivants peuvent être avantageusement mis en oeuvre dans la présente invention : Rhodiastab 50®, Rhodiastab X5®, Rhodiastab X2®, Rhodiastab 83®, commercialisés par Rhodia Chimie.

Les composés sous forme de chélates sont aussi des produits connus et l'on peut accéder à ces composés par réaction de la β-dicétone concernée avec des sels des métaux précités, comme notamment les chlorures, sulfates, nitrates, avec des oxydes ou hydroxydes, avec le métal lui-même, avec des carbonates ou encore avec des alcoxydes. Il est à noter que ces méthodes sont notamment décrites dans l'ouvrage *"Metal β -diketonates and allied derivatives" de R.C. Mehrota, R. Gaur, D.P. Gaur, paru* en 1978, Academic Press).

Les chélates de l'octanoylbenzoylméthane, du stéaroylbenzoylméthane, du dibenzoylméthane, de l'acétylbenzoylméthane ou encore de l'acétylacétone (à l'exception des acétylacétonates de calcium et de mégnasium), seuls ou en mélange, peuvent être utilisés avantageusement.

La teneur totale en composé β-dicétonique, libre et/ou sous forme de chélate, est comprise entre 0,05 et 1 g pour 100 g de polymère halogéné. De préférence, la teneur en composé β-dicétonique est comprise entre 0,1 et 0,5 g pour 100 g de polymère halogéné.

Ainsi, les formulations à base de polymère halogéné peuvent comprendre au moins un composé capteur d'acide chlorhydrique.

Les composés capteurs d'acide chlorhydrique peuvent être de type organique ou de type minéral, et peuvent être présents seuls ou en mélanges.

Parmi les capteurs d'acide chlorhydrique de type organique, on peut citer plus particulièrement les composés comprenant un métal alcalino-terreux ou un métal choisi dans les colonnes IIB, IIA, IVB de la classification périodique des éléments (parue dans le supplément au Bulletin de la Société Chimique de France, no. 1, janvier 1966).

Les cations sont plus particulièrement de préférence choisis parmi le calcium, le baryum, le magnésium, le strontium, le zinc, le cadmium, l'étain ou encore le plomb.

Il est à noter que des associations sont envisageables comme par exemple un mélange de capteur d'acide chlorhydrique à base de calcium et de zinc, de baryum et de zinc, de baryum et de cadmium, la première association étant préférée.

En ce qui concerne les composés capteurs d'acide chlorhydrique de type organique comprenant au moins l'un des éléments des colonnes IIB et IIA, on peut citer tout particulièrement les sels d'acides organiques, tels que les acides carboxyliques aliphatiques, aromatiques ou les acides gras, ou encore les phénolates ou les alcoolates aromatiques.

Les plus couramment utilisés sont par exemple les sels des éléments IIA ou IIB des acides maléique, acétique, diacétique, propionique, hexanoïque, éthyl-2 hexanoïque, décanoïque, undécanoïque, laurique, myristique, palmitique, stéarique, oléïque, ricinoléïque, béhénique (docosanoïque), hydroxystéarique, hydroxy-undécanoïque, benzoïque, phénylacétique, paratertiobutylbenzoïque et salicylique, les phénolates, les alcoolates dérivés du naphtol ou des phénols substitués par un ou plusieurs radicaux alkyle, tels que les nonylphénols.

Pour des raisons pratiques ou pour des raisons économiques, on choisit de préférence parmi les composés organiques du métal alcalino-terreux cités précédemment, le propionate de métal alcalino-terreux, l'oléate de métal alcalino-terreux, le stéarate de métal alcalino-terreux, le laurate de métal alcalino-terreux, le ricinoléate de métal alcalino-terreux, le docosanoate de métal alcalino-terreux, le benzoate de métal alcalino-terreux, le paratertiobutylbenzoate de métal alcalino-terreux, le salicylate de métal alcalino-terreux, le maléate de métal alcalino-terreux et de mono-éthyl-2 hexyle), les nonylphénates de métal alcalino-terreux, le naphténate de métal alcalino-terreux et parmi les composés organiques du cadmium cités précédemment, le propionate de cadmium, l'éthyl-2 hexanoate de cadmium, le laurate de cadmium, le stéarate de cadmium, le salicylate de cadmium, le maléate de cadmium et de mono(éthyl-2 hexyle), les nonylphénates de cadmium, le naphténate de cadmium.

En ce qui concerne les composés de type organique comprenant du plomb, on peut citer notamment ceux décrits dans *ENCYCLOPEDIA of PVC de Leonard I. NASS (1976) page 299-303.*

Ce sont des composés très divers dont les plus couramment utilisés sont le carbonate dibasique de plomb, le sulfate tribasique de plomb, le sulfate tétrabasique de plomb, le phosphite dibasique de plomb, l'orthosilicate de plomb, le silicate basique de plomb, le coprécipitat de silicate et de sulfate de plomb, le chlorosilicate basique de plomb, le coprécipitat de gel de silice et d'orthosilicate de plomb, le phatalate dibasique de plomb, le stéarate neutre de plomb, le stéarate dibasique de plomb, le fumarate tétrabasique de plomb, le maléate dibasique de plomb, l'éthyl-2 hexanoate de plomb, le laurate de plomb.

Pour ce qui a trait aux composés à base d'étain, on peut notamment se reporter à l'ouvrage *"PLASTICS ADDITIVES HANDBOOK" de GACHTER*/*MULLER (1985) pages 204-210 ou dans ENCYCLOPEDIA OF PVC de Léonard I. NASS (1976) pages 313-325.*

Ce sont plus particulièrement des carboxylates de mono- ou di-alkylétain et des mercaptides de mono- ou di-alkylétain.

Parmi ces composés les plus couramment utilisés sont les dérivés de di-n-méthylétain, de di-n-butylétain ou de di-n-octylétain tels que par exemple le dilaurate de dibutylétain,le maléate de dibutylétain, le laurate-maléate de dibutylétain, le bis(maléate de mono-C₄-C₈-alkyle) de dibutylétain, le bis(lauryl-mercaptide) de dibutylétain, le S-S' (mercatoacétate d'isooctyle) dibutylétain, le β-mercapto propionate de dibutylétain, le maléate de di-n-octylétain polymère, le bis-S-S'(mercaptoacétate d'isooctyle)di-n-octylétain, le β-mercapto-propionate de di-n-octylétain. Les dérivés monoalkylés des composés cités ci-dessus sont aussi convenables.

Comme capteur d'acide chlorhydrique de type minéral, on peut aussi citer les sulfates, et/ou les carbonates, d'aluminium et/ou de magnésium, du type hydrotalcite notamment. Il est rappelé que les composés du type hydrotalcite correspondent à la formule suivante : Mg₁₋ₓAlₓ(OH)₂Aⁿ⁻_{x/n}. mH₂O, dans laquelle x est compris entre 0 exclu et 0,5, Aⁿ⁻ représente un anion tel que le carbonate notamment, n varie de 1 à 3 et m est positif. Il est à noter que l'on peut mettre en oeuvre des produits de ce type, ayant subi un traitement de surface avec un composé organique. On ne sortirait de même pas du cadre de la présente invention en mettant en oeuvre un produit du type hydrotalcite dopé par du zinc, ayant éventuellement subi un traitement de surface par un composé organique. Parmi les produits de ce type, on peut citer tout particulièrement l'Alcamizer® 4 (commercialisé par la société Kyowa).

On peut aussi utiliser des composés essentiellement amorphes de formule (MgO)_{y}, Al₂O₃, (CO₂)ₓ, (H₂O)_{z}, dans laquelle x, y et z vérifient les inéquations suivantes : 0 < x ≤ 0,7 ; 0 < y ≤ 1,7 et z ≥ 3. Ces composés sont notamment décrits dans la demande de brevet EP 509 864. Par ailleurs, les composés appelés catoïtes de formule Ca₃Al₂(OH)₁₂ ou encore Ca₃Al₂(SiO)₄(OH)₁₂ conviennent en tant que composés capteur d'acide chlorhydrique de type minéral.

Ainsi, les formulations à base de polymères halogénés peuvent comprendre du dioxyde de titane.

De préférence, le dioxyde de titane est sous le forme rutile.

Généralement, la granulométrie du dioxyde de titane entrant dans les compositions stabilisantes selon l'invention, est comprise entre 0,1 et 0,5 µm.

Selon un mode de réalisation particulier de l'invention, on utilise du dioxyde de titane sous forme rutile ayant subi un traitement de surface, de préférence minéral.

Parmi les dioxydes de titane convenant particulièrement bien à la mise en oeuvre de la présente invention, on peut citer sans intention de s'y limiter, le dioxyde de titane Rhoditan® RL18, Rhoditan® RL90, commercialisés par Rhodia Chimie, les dioxydes de titane Kronos 2081® et 2220® commercialisés par Kronos.

Les formulations à base de polymères halogénés peuvent comprendre de même d'autres pigments blancs ou colorés. Parmi les pigments colorés, on peut citer notamment le sulfure de cérium.

Il est à noter que la quantité de pigment introduite dans la formulation varie dans de larges limites et dépend notamment du pouvoir colorant du pigment et de la coloration finale souhaitée. Cependant, à titre d'exemple, la quantité de pigment peut varier de 0,1 à 20 g pour 100 g de polymère halogéné, de préférence de 0,5 à 15 g par rapport à la même référence.

La formulation peut en outre comprendre au moins un polyol comprenant 2 à 32 atomes de carbone et présentant deux à neuf groupements hydroxyles.

Parmi ces composés on peut mentionner les diols en C₃-C₃₀ tels que le propylène glycol, le butanediol, l'hexanediol, le dodécanediol, le néopentylglycol, les polyols tels que le triméthylolpropane, le pentaérythritol, le dipentaérythritol, le tripentaérythritol, le xylitol, le mannitol, le sorbitol, la glycérine, les mélanges d'oligomères de la glycérine présentant un degré de polymérisation de 2 à 10.

Une autre famille de polyols pouvant être convenablement mise en oeuvre, est constituée par les alcools polyvinyliques partiellement acétylés.

On peut de même utiliser des composés hydroxylés comprenant des groupements isocyanurates, seuls ou en combinaison avec les polyols précités, tels que par exemple le tris (2-hydroxyéthyl) isocyanurate.

La quantité de polyol mise en oeuvre est en général comprise entre 0,05 et 5 g pour 100 g de polymère halogéné. Plus particulièrement elle est inférieure à 2 g pour 100 g de polymère halogéné.

On peut éventuellement incorporer dans la formulation des composés du type des phosphites organiques, comme par exemple, les phosphites de trialkyle, d'aryle, de triaryle, de dialkylaryle, ou de diarylalkyle, pour lesquels le terme alkyle désigne des groupements hydrocarbonés de monoalcools ou de polyols en C₈-C₂₂, et le terme aryle désigne des groupements aromatiques de phénol ou de phénol substitué par des groupements alkyles en C₆-C₁₂. On peut de même utiliser des phosphites de calcium, comme par exemple des composés du type Ca(HPO₃).(H₂O) ainsi que des complexes phosphite - hydroxy - aluminium - calcium.

La teneur en additif de ce type est habituellement comprise entre 0,1 et 2 g pour 100 g de polymère halogéné.

Les formulations peuvent de même comprendre au moins un aluminosilicate de métal alcalin, cristallin, synthétique, présentant une teneur en eau comprise entre 13 et 25 % en poids, de composition 0,7-1M₂O.Al₂O₃.1,3-2,4SiO₂ dans laquelle M représente un métal alcalin tel que notamment le sodium. Conviennent notamment les zéolites de type NaA, telles que décrites dans le brevet US 4 590 233.

La teneur en ce type de composés varie généralement entre 0,1 et 5 g pour 100 g de polymère halogéné.

Les formulations peuvent aussi comprendre des composés du type des époxydes. Ces composés sont généralement choisis parmi les polyglycérides époxydés, ou les esters d'acides gras époxydés, tels que les huiles époxydées de lin, de soja ou de poisson.

La quantité de composés de ce type varie habituellement entre 0,5 et 10 g pour 100 g de polymère halogéné.

D'autres additifs classiques peuvent compléter la formulation, selon l'application à laquelle elle est destinée.

En règle générale, la formulation peut comprendre des antioxydants phénoliques, des agents anti-UV tels que les 2-hydroxybenzophénones, les 2-hydroxybenzotriazoles ou les amines stériquement encombrées, connues habituellement sous le terme Hals.

La teneur en ce type d'additif varie généralement entre 0,05 et 3 g pour 100 g de polymère halogéné.

Si nécessaire, on peut aussi utiliser des lubrifiants qui vont faciliter la mise en oeuvre, choisis notamment parmi les monostéarates de glycérol ou encore le propylène glycol, les acides gras ou leurs esters, les cires montanates, les cires de poyléthylène ou leur dérivés oxydés, les paraffines, les savons métalliques, les huiles polyméthylsiloxanes fonctionnalisées comme par exemple les huiles γ-hydroxypropylénées.

La quantité de lubrifiant entrant dans la formulation à base de polymère halogéné varie en général entre 0,05 et 2 g pour 100 g de polymère halogéné.

La formulation peut aussi comprendre des plastifiants choisis parmi les phtalates d'alkyle. Les composés les plus généralement utilisés sont choisis parmi le phtalate de di (éthyl- 2 - hexyle), les esters de diacides linéaires en C₆-C₁₂, les triméllitates ou encore les phosphates esters.

La quantité d'agent plastifiant employée dans les formulations, varie dans un large domaine, en fonction du caractère rigide ou souple du polymère final. A titre indicatif, la teneur varie de 0 à 100 g pour 100 g de polymère.

La préparation des formulations peut être faite par tout moyen connu de l'homme du métier.

On peut ainsi incorporer les divers constituants au polymère individuellement ou bien après avoir préparé préalablement un mélange de plusieurs de ces constituants, comme par exemple, la composition stabilisante de l'invention seule ou en présence de lubrifiant.

Les méthodes classiques d'incorporation conviennent parfaitement à l'obtention de la formulation à base de PVC.

Ainsi, et seulement à titre indicatif, on peut effectuer cette opération dans un mélangeur muni d'un système de pâles et de contre-pales fonctionnant à une vitesse élevée.

Généralement, l'opération de mélange est conduite à une température inférieure à 130°C.

Une fois le mélange réalisé, on effectue une mise en forme de la composition selon les méthodes habituelles dans le domaine comme l'injection, l'extrusion-soufflage, l'extrusion, le calandrage ou encore le moulage par rotation.

La température à laquelle est réalisée la mise en forme varie en général de 150 à 220°C.

Des exemples concrets mais non limitatifs de l'invention vont maintenant être présentés.

### EXEMPLE 1

Cet exemple a pour objet de préparer l'acétylacétonate de calcium enrobé.

### 1/ Préparation d'acétylacétonate de calcium enrobé par l'acide stéarique (E1)

On prépare une solution enrobante en ajoutant 3 g d'acide stéarique dans 150 ml d'hexane en chauffant le mélange à 55°C, et sous agitation.

On ajoute à cette solution, 57 g d'acétylacétonate de calcium. L'opération a lieu sous agitation et à pression atmosphérique, à température ambiante.

Le mélange est agité pendant 45 minutes.

Le séchage a lieu comme suit :
* élimination progressive et régulière de la majeure partie du solvant (350 mbar, 55-60 °C) ;
* séchage à 55-60°C à une pression de 10 à 15 mbar ;
* la masse réactionnelle solide résultante est récupérée, broyée et séchée sous un vide de 10 mbar à une température de 55-60°C.

### 2/ Préparation d'acétylacétonate de calcium enrobé par une huile silicone (E2)

On prépare une solution enrobante en ajoutant 3 g d'acide stéarique dans 200 ml d'hexane en chauffant le mélange à 60°C, et sous agitation.

On ajoute à cette solution, 57 g d'acétylacétonate de calcium.

L'opération a lieu sous agitation et à pression atmosphérique, à 60°C.

Le mélange est agité pendant 2 heures.

Le séchage a lieu comme suit :
* élimination progressive et régulière de la majeure partie du solvant (450 mbar, 60 °C) ;
* séchage à 60°C à une pression 50 mbar ;
* la masse réactionnelle solide résultante est récupérée, broyée et séchée sous un vide de 10 mbar à une température de 60°C.

### EXEMPLE 2

Cet exemple a pour objet d'évaluer l'état de dispersion de l'acétylacétonate de calcium enrobé ou non.

On prépare trois échantillons à partir de la formulation dont la composition est indiquée dans le tableau ci-dessous :

| | |
|---|---|
| * Résine PVC préparée par polymérisation en suspension et commercialisée sous la dénomination S110P® (Atochem) | 100 parties |
| * TiO₂ (Kronos 2220®) | 6,0 parties |
| * Stabilisant : hydroxystéarate de calcium Stavinor® (Atochem) stéarate de zinc ZN70® (Atochem) | 0,3 partie 1 partie |
| * Hydrotalcite Alcamizer 4® (Mitsui) | 0,6 partie |
| * Alcool polyvinylique Polyol PVAL® | 0,2 partie |
| * CaCO₃ Hydrocarb 95 T® (Omya) | 5,0 parties |
| * Renforçant choc - polymère acrylique Paraloid KM 355® (Rohm & Haas) | 6,5 parties |
| * Lubrifiants : Loxiol G 60® (Henkel) Loxiol G 22® (Henkel) | 0,4 partie 0,2 partie |
| * Agent de mise en oeuvre (processing aid) Paraloid K120N® (Rohm & Haas) | 1 partie |

Dans le premier échantillon **E0 comparatif**, on introduit de l'acétylacétonate de calcium à raison de 0,3 partie pour 100 parties de résine PVC.

Dans le deuxième échantillon **E1 selon l'invention**, obtenu précédemment, on introduit de l'acétylacétonate de calcium (0,3 parties pour 100 parties de résine PVC) enrobé par l'acide stéarique.

Dans le troisième échantillon **E2 selon l'invention**, obtenu précédemment, on introduit de l'acétylacétonate de calcium (0,3 parties pour 100 parties de résine PVC), enrobé par l'huile silicone.

Le mélange des poudres est effectué dans un mélangeur rapide du typé Papen Meier (vitesse de rotation de 2500 tr/min). L'opération de mélange est stoppée une fois que la température du mélange atteint 113-115°C.

A partir de ce mélange de poudres, on procède à une transformation par extrusion afin d'obtenir des plaques.

Les caractéristiques de l'extrudeuse bivis sont :
- fabriquant Brabender
- vis parallèle : rapport longueur / diamètre : 42 / 6 D SK
- filière plate.

Les conditions d'extrusion pour l'obtention des profilés sont :
- vitesse de rotation de la vis ; 20 tr/min
- profil de température :

| | | |
|---|---|---|
| zone 1 | zone 2 | zone 3 |
| 175°C | 185°C | 185°C |

On mesure alors l'indice de jaune (coefficient b) des plaques extrudées obtenues.

Le coefficient b est l'un des paramètres du système (L, a, b) CIE. Il est rappelé que les mesures de l'indice a sont faites sur des plaques extrudées, à l'aide d'un chromomètre-colorimètre MINOLTA CR 200®

Les résultats sont rassemblés dans le tableau ci-dessous :

| **ECHANTILLON** | **COEFFICIENT b** |
|---|---|
| E0 | 3,6 |
| E1 | 3,4 |
| E2 | 3,3 |

Il est à noter que l'indice de jaune est le reflet de l'état de dispersion de l'acétylacétonate de calcium dans la formulation polymérique.

Ainsi, l'amélioration de cet indice (c'est-à-dire la diminution de la valeur du coefficient) indique une meilleure dispersion.

On constate donc que les échantillons E1 et E2 selon l'invention sont mieux dispersés que l'échantillon E0 comparatif.

### EXEMPLE 3

Cet exemple a pour objet de préparer l'acétylacétonate de calcium enrobé par de l'alcool stéarique.

On prépare une solution enrobante en ajoutant 2,5 g d'alcool stéarique dans 120 ml d'hexane en chauffant le mélange à 60°C, et sous agitation.

On ajoute à cette solution, 47,5 g d'acétylacétonate de calcium.

L'opération a lieu sous agitation, à pression atmosphérique et à température ambiante.

Le mélange est agité pendant 45 minutes.

Le séchage a lieu comme suit :
* élimination progressive et régulière de la majeure partie du solvant (330 mbar, 55-60 °C) ;
* séchage à 55-60°C à une pression 8-10 mbar.

### EXEMPLE 4

Cet exemple a pour objet d'évaluer l'état de dispersion de l'acétylacétonate de calcium enrobé ou non.

### 1/ Préparation d'un mélange maître noir

La composition est la suivante :

| | |
|---|---|
| * Résine PVC Lacovyl GV 13/10® (Solvay) | 100 parties |
| * Stéarate de calcium | 0,25 partie |
| * Noir de carbone | 0,25 partie |
| * Dioctylphtalate | 29 parties |
| * Tinstab BM271® (Ackros Chemicals) | 0,2 partie |
| * Lubrifiant | 0,5 partie |

On mélange les poudres dans un malaxeur Hobart® (type Kenwood planétaire) pendant 30 minutes.

On ajoute ensuite les composés liquides sous agitation, à une température de 50°C, en 30 minutes.

On maintient l'agitation pendant 1 heure à 50 °C.

### 2/ Calandrage

Le mélange maître noir obtenu auparavant est mis en oeuvre, avec l'acétylacétonate de calcium, d'une part sous la forme d'un échantillon d'acétylacétonate de calcium enrobé obtenu à l'exemple 3 (**E3 selon l'invention**), d'autre part, sous la forme d'un échantillon d'acétylacétonate de calcium seul (**E0 comparatif**), sur un mélangeur à cylindres Troester®.

### (a) caractéristiques de l'appareil :

Mélangeur à deux cylindres Troester type WNK 1 n° 1355
Cylindres : diamètre : 101 mm ; longueur : 250 mm.
   Les cylindres tournent à une vitesse de 29 tr/mn ;
   Le rapport de friction est de 1/1 (coefficient de friction nul) ;
   La température sur cylindre est de 175°C..

### (b) mode opératoire

On gélifie 100 g de mélange maître noir obtenu au point 1/ sur le mélangeur Troester.

Après 90 secondes de calandrage, l'écartement des cylindres étant alors réglé à 0,7 (1 mm d'épaisseur de feuille), on ajoute 2,5 g d'acétylacétonate (d'une part, sous forme d'un échantillon E0, d'autre part, sous forme d'un échantillon E3).

On réalise enfin un passage "au fin" avec un écartement des cylindres de 0,4.

Après 210 secondes de calandrage, on sort une feuille de 1 mm d'épaisseur (écartement des cylindres à 0,7) et on refroidit les plaques obtenues.

Les feuilles calandrées sont comparées visuellement. Le nombre de points blancs apparaissant sur le fond noir de la plaque caractérise l'état de dispersion de l'acétylacétonate de calcium.

Le nombre de piqûres ou agglomérats visibles est moins important dans le cas de la plaque comprenant l'échantillon E3, par rapport à la plaque comprenant l'échantillon E0, démontrant une meilleure dispersion du composé enrobé selon l'invention dans la formulation polymérique.

## Revendications

1. Particules comprenant de l'acétylacétonate de calcium ou de magnésium, ou leur combinaison, partiellement ou totalement enrobé par au moins un agent compatibilisant choisi parmi :
□ les alcools comprenant 12 à 30 atomes de carbone, saturés ou non ;
□ les acides carboxyliques ou sulfoniques, comprenant 12 à 30 atomes de carbone, saturés ou non, substitués ou non par au moins un groupement hydroxyle, ou leurs dérivés ;
□ les phosphates ou les titanates comprenant au moins une chaîne comprenant 12 à 30 atomes de carbone, saturée ou non ;
□ les composés β-dicétoniques présentant au moins une chaîne comprenant au moins 7 atomes de carbone ;
□ les cires ;
□ les polyols ;
□ les huiles végétales époxydées ;
□ les huiles ou les résines polysiloxaniques ou encore les silanes.

2. Particules selon la revendication précédente, **caractérisées en ce que** l'agent compatibilisant est un alcool choisi parmi les monoalcools aliphatiques, saturés ou non, comprenant 12 à 30 atomes de carbone.

3. Particules selon la revendication précédente, **caractérisées en ce que** l'alcool est choisi parmi les alcools laurique, myristique, stéarique, isostéarique, cétylique, behénique, lauroléique, oléique, érucique, linoléique.

4. Particules selon la revendication 1, **caractérisées en ce que** l'agent compatibilisant est choisi parmi les acides stéarique, laurique, myristique, palmitique, oléïque, ricinoléïque, béhénique (docosanoïque), l'acide linoléique, l'acide linolénique, l'acide ricinoléique, l'acide hydroxystéarique, ou tout autre acide provenant des glycérides ou triglycérides, naturels ou non.

5. Particules selon la revendication 1, **caractérisées en ce que** l'agent compatibilisant est un dérivé des acides choisi parmi les esters des acides carboxyliques précités, obtenus à partir de monoalcools comprenant 1 à 30 atomes de carbone, ou les mono- ou polyesters obtenus à partir de polyols.

6. Particules selon la revendication 1, **caractérisées en ce que** l'agent compatibilisant est un dérivé des acides choisi parmi les sels de métaux alcalins, alcalino-terreux, d'aluminium, de lanthane et de zinc des acides carboxyliques précités.

7. Particules selon la revendication 1, **caractérisées en ce que** l'agent compatibilisant est l'acide dodécylbenzène sulfonique.

8. Particules selon la revendication 1, **caractérisées en ce que** l'agent compatibilisant est un composé β-dicétonique de formule R¹COCHR²COR³ ; dans laquelle le radical R¹, représente un radical hydrocarboné, linéaire ou ramifié, substitué
ou non, en C₇-C₃₀, le radical R³ représente un radical hydrocarboné, linéaire ou ramifié, substitué ou non, en C₁-C₃₀, le radical R² représente un atome d'hydrogène ou un radical hydrocarboné, linéaire ou ramifié, en C₁-C₄.

9. Particules selon la revendication 1, **caractérisées en ce que** l'agent compatibilisant est choisi parmi les cires comme les cires montanates, les cires de poyléthylène ou leur dérivés oxydés, ainsi que les paraffines.

10. Particules selon la revendication 1, **caractérisées en ce que** l'agent compatibilisant est choisi parmi les polyols comprenant 2 à 32 atomes de carbone, présentant deux à neuf groupements hydroxyles, et dont les groupements hydroxyles se trouvent indifféremment en position vicinale ou non, ou bien les alcools polyvinyliques, ou encore des polyols comprenant des groupements isocyanurates.

11. Particules selon la revendication précédente, **caractérisées en ce que** le polyol est choisi parmi le propylène glycol, butylèneglycol, le butanediol, pentanediol, l'hexanediol, le dodécanediol, le néopentylglycol, les polyols tels que le triméthylolpropane, le pentaérythritol, le dipentaérythritol, le tripentaérythritol, le xylitol, le mannitol, le sorbitol, la glycérine, les mélanges d'oligomères de la glycérine présentant un degré de polymérisation de 2 à 10, l'alcool hydroxystéarique, l'alcool ricinoléique.

12. Particules selon la revendication 1, **caractérisées en ce que** l'agent compatibilisant est une huile végétale époxydée telle que l'huile de soja époxydée, l'huile de ricin époxydée.

13. Particules selon la revendication 1, **caractérisées en ce que** l'agent compatibilisant est une huile polysiloxanique correspondant à la formule générale suivante : YO-[(R)Si(R)-O]ₓ-Y, formule dans laquelle R, identiques ou différents, représentent un radical alkyle comprenant 1 à 3 atomes de carbone, et de préférence un méthyle, ou un atome d'hydrogène à la condition que seulement l'un des deux radicaux soit un hydrogène, Y représente un atome d'hydrogène ou (R')₃Si, avec R', identiques ou différents, représentant un radical alkyle comprenant 1 à 3 atomes de carbone, de préférence le méthyle, avec x variant entre 5 et 300.

14. Particules selon la revendication précédente, **caractérisées en ce que** l'agent compatibilisant est une huile polysiloxanique choisie parmi les huiles polyméthylsiloxanes fonctionnalisées, comme les huiles γ-hydroxypropylénées.

15. Particules selon la revendication 1, **caractérisées en ce que** l'agent compatibilisant est choisi parmi les résines polysiloxaniques obtenues par l'action d'huiles polysiloxaniques portant des groupements vinyles, en présence d'un catalyseur à base de platine, ou bien celles obtenues par hydrolyse et auto-condensation d'au moins un silane de formule (RO)₃SiF,ou (RO)₂(Me)SiF, dans lesquelle R, identiques ou différents, représentent un radical alkyle comprenant 1 à 4 atomes de carbone, F représente plus particulièrement les radicaux suivants :
-CH=CH₂,
-(CH₂)₃OH, -(CH₂)₃-NH₂, -(CH₂)₃NHCH₂CH₂NH₂, 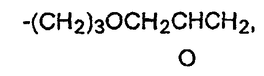
-(CH₂)₃O-CO-CH=CH₂, -(CH₂)₃O-CO-(CH₃)CH=CH₂,
ou parmi les silanes précités en tant que tel.

16. Particules selon l'une quelconque des revendications précédentes, **caractérisées en ce que** la proportion en poids d'agent compatibilisant par rapport à l'acétylacétonate de calcium ou de magnésium est comprise entre 0,1 et 20 % en poids par rapport au poids d'acétylacétonate de calcium ou de magnésium, plus particulièrement, entre 0,1 et 10 % en poids par rapport au poids d'acétylacétonate de calcium ou de magnésium.

17. Procédé de préparation de particules selon l'une quelconque des revendications précédentes, **caractérisé en ce que** l'on met en contact l'acétylacétonate précité avec au moins un agent compatibilisant, en présence d'un solvant et/ou d'un dispersant.

18. Procédé selon la revendication précédente, **caractérisé en ce que** le solvant et/ou dispersant est choisi parmi l'eau, les monoalcools en C₁-C₅, notamment tels que le méthanol, l'éthanol, les éthers en C₂-C₆ comme le diméthyléther, le méthyléthyléther, le diéthyléther, les hydrocarbures comme entre autres l'hexane.

19. Utilisation de particules selon l'une quelconque des revendications 1 à 16, comme additif dans des formulations comprenant au moins un polymère halogéné.

20. Utilisation selon la revendication précédente, **caractérisée en ce que** la teneur en particules, exprimée en acétylacétonate de calcium ou de magnésium, est comprise entre 0,01 et 5 g pour 100 g de polymère halogéné, plus particulièrement, entre 0,05 et 2 g par rapport à la même référence.

## Patentansprüche

1. Teilchen, umfassend Calciumacetylacetonat oder Magnesiumacetylacetonat oder deren Kombination, teilweise oder vollständig umhüllt durch mindestens ein Kompatibilisierungsmittel, ausgewählt unter:
· den gesättigten oder ungesättigten Alkoholen mit 12 bis 30 Kohlenstoffatomen;
· den gesättigten oder ungesättigten Carbonsäuren oder Sulfonsäuren mit 12 bis 30 Kohlenstoffatomen, unsubstituiert oder substituiert durch mindestens eine Hydroxylgruppe, oder ihren Derivaten;
· den Phosphaten oder Titanaten, umfassend mindestens eine gesättigte oder ungesättigte Kette mit 12 bis 30 Kohlenstoffatomen;
· den β-diketonischen Verbindungen, die mindestens eine Kette mit mindestens 7 Kohlenstoffatomen aufweisen;
· den Wachsen;
· den Polyolen;
· den epoxidierten Pflanzenölen;
· den Polysiloxan-Ölen oder den Polysiloxan-Harzen oder auch den Silanen.

2. Teilchen nach dem vorstehenden Anspruch, **dadurch gekennzeichnet, daß** das Kompatibilisierungsmittel ein Alkohol ist, ausgewählt unter den gesättigten oder ungesättigten aliphatischen Monoalkoholen mit 12 bis 30 Kohlenstoffatomen.

3. Teilchen nach dem vorstehenden Anspruch, **dadurch gekennzeichnet, daß** der Alkohol ausgewählt wird unter Laurinalkohol, Myristinalkohol, Stearinalkohol, Isostearinalkohol, Cetylalkohol, Behenalkohol, Lauroleinalkohol, Oleinalkohol, Erucaalkohol, Linolalkohol.

4. Teilchen nach Anspruch 1, **dadurch gekennzeichnet, daß** das Kompatibilisierungsmittel ausgewählt wird unter Stearinsäure, Laurinsäure, Myristinsäure, Palmitinsäure, Oleinsäure, Ricinoleinsäure, Behensäure (Docosanoesäure), Linolsäure, Linolensäure, Ricinoleinsäure, Hydroxystearinsäure, oder jeder anderen Säure, die von natürlichen oder nicht natürlichen Glyceriden oder Triglyceriden stammt.

5. Teilchen nach Anspruch 1, **dadurch gekennzeichnet, daß** das Kompatibilisierungsmittel ein Derivat von Säuren ist, ausgewählt unter den Estern der vorstehend genannten Carbonsäuren, erhalten ausgehend von Monoalkoholen mit 1 bis 30 Kohlenstoffatomen, oder den Mono- oder Polyestern, erhalten ausgehend von Polyolen.

6. Teilchen nach Anspruch 1, **dadurch gekennzeichnet, daß** das Kompatibilisierungsmittel ein Derivat von Säuren ist, ausgewählt unter den Salzen von Alkalimetallen, Erdalkalimetallen, Aluminium, Lanthan und Zink der vorstehend genannten Carbonsäuren.

7. Teilchen nach Anspruch 1, **dadurch gekennzeichnet, daß** das Kompatibilisierungsmittel Dodecylbenzolsulfonsäure ist.

8. Teilchen nach Anspruch 1, **dadurch gekennzeichnet, daß** das Kompatibilisierungsmittel eine β-diketonische Verbindung der Formel R¹COCHR²COR³ ist, worin der Rest R¹ einen geraden oder verzweigten, substituierten oder unsubstituierten Kohlenwasserstoff-Rest mit 7 bis 30 Kohlenstoffatomen darstellt, der Rest R³ einen geraden oder verzweigten, substituierten oder unsubstituierten Kohlenwasserstoff-Rest mit 1 bis 30 Kohlenstoffatomen bedeutet und der Rest R² ein Wasserstoffatom oder einen geraden oder verzweigten Kohlenwasserstoff-Rest mit 1 bis 4 Kohlenstoffatomen darstellt.

9. Teilchen nach Anspruch 1, **dadurch gekennzeichnet, daß** das Kompatibilisierungsmittel ausgewählt wird unter den Wachsen wie den Montanatwachsen, den Polyethylenwachsen oder ihren oxidierten Derivaten, sowie den Paraffinen.

10. Teilchen nach Anspruch 1, **dadurch gekennzeichnet, daß** das Kompatibilisierungsmittel ausgewählt wird unter den Polyolen mit 2 bis 32 Kohlenstoffatomen, die zwei bis neun Hydroxylgruppen aufweisen und deren Hydroxylgruppen sich unterschiedslos in vicinaler oder nicht vicinaler Position befinden, oder den Polyvinylalkoholen oder auch den Polyolen mit Isocyanurat-Gruppen.

11. Teilchen nach dem vorstehenden Anspruch, **dadurch gekennzeichnet, daß** das Polyol ausgewählt wird unter Propylenglycol, Butylenglycol, Butandiol, Pentandiol, Hexandiol, Dodecandiol, Neopentylglycol, den Polyolen wie Trimethylolpropan, Pentaerythritol, Dipentaerythritol, Tripentaerythritol, Xylitol, Mannitol, Sorbitol, Glycerin, Mischungen von Oligomeren des Glycerins, die einen Polymerisationsgrad von 2 bis 10 aufweisen, Hydroxystearinalkohol, Ricinoleinalkohol.

12. Teilchen nach Anspruch 1, **dadurch gekennzeichnet, daß** das Kompatibilisierungsmittel ein epoxidiertes Pflanzenöl ist, wie epoxidiertes Sojaöl und epoxidiertes Rizinusöl.

13. Teilchen nach Anspruch 1, **dadurch gekennzeichnet, daß** das Kompatibilisierungsmittel ein Polysiloxanöl ist, das der folgenden allgemeinen Formel entspricht: YO-[(R)Si(R)-O)]ₓ-Y, in der R, gleich oder verschieden, einen Rest Alkyl mit 1 bis 3 Kohlenstoffatomen, vorzugsweise ein Methyl, oder ein Wasserstoffatom darstellt, unter der Bedingung, daß nur einer der beiden Reste Wasserstoff ist, Y ein Wasserstoffatom oder (R')₃Si bedeutet, worin R', gleich oder verschieden, einen Rest Alkyl mit 1 bis 3 Kohlenstoffatomen, vorzugsweise Methyl darstellt, und x zwischen 5 und 300 variieren kann.

14. Teilchen nach dem vorstehenden Anspruch, **dadurch gekennzeichnet, daß** das Kompatibilisierungsmittel ein Polysiloxanöl ist, ausgewählt unter den funktionalisierten Polymethylsiloxanölen wie den γ-hydroxypropylenierten Ölen.

15. Teilchen nach Anspruch 1, **dadurch gekennzeichnet, daß** das Kompatibilisierungsmittel ausgewählt wird unter den Polysiloxanharzen, erhalten durch Einwirkung von Polysiloxanölen, die Vinylgruppen tragen, in Anwesenheit eines Katalysators auf der Basis von Platin, oder unter denen, die erhalten werden durch Hydrolyse und Auto-Kondensation von mindestens einem Silan der Formel (RO)₃SiF oder (RO)₂(Me)SiF, worin R, gleich oder verschieden, einen Rest Alkyl mit 1 bis 4 Kohlenstoffatomen darstellt und F ganz besonders die folgenden Reste bedeutet:
―CH=CH₂,
―(CH₂)₃OH, ―(CH₂)₃―NH₂, ―(CH₂)₃NHCH₂CH₂NH₂, 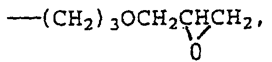
―(CH₂)₃O―CO―CH=CH₂,―(CH₂)₃O―CO―(CH₃)CH=CH₂,
oder unter den vorstehend genannten Silanen als solche.

16. Teilchen nach irgendeinem der vorstehenden Ansprüche, **dadurch gekennzeichnet, daß** das Gewichtsverhältnis des Kompatibilisierungsmittels, bezogen auf Calciumacetylacetonat oder Magnesiumacetylacetonat, zwischen 0,1 Gew.-% und 20 Gew.-%, bezogen auf das Gewicht von Calciumacetylacetonat oder Magnesiumacetylacetonat, und ganz besonders zwischen 0,1 Gew.-% und 10 Gew.-% beträgt, bezogen auf das Gewicht von Calciumacetylacetonat oder Magnesiumacetylacetonat.

17. Verfahren zur Herstellung der Teilchen nach irgendeinem der vorstehenden Ansprüche, **dadurch gekennzeichnet, daß** man das vorstehend genannte Acetylacetonat mit mindestens einem Kompatibilisierungsmittel in Anwesenheit eines Lösungsmittels und/oder eines Dispergierungsmittel in Kontakt bringt.

18. Verfahren nach dem vorstehenden Anspruch, **dadurch gekennzeichnet, daß** das Lösungsmittel und/oder das Dispergierungsmittel ausgewählt wird unter Wasser, den Monoalkoholen mit 1 bis 5 Kohlenstoffatomen, insbesondere Methanol, Ethanol, den Ethern mit 2 bis 6 Kohlenstoffatomen, wie Dimethylether, Methylethylether, Diethylether, den Kohlenwasserstoffen wie unter anderem Hexan.

19. Verwendung der Teilchen nach irgendeinem der Ansprüche 1 bis 16 als Zusatzstoff in Formulierungen, die mindestens ein halogeniertes Polymer umfassen.

20. Verwendung nach dem vorstehenden Anspruch, **dadurch gekennzeichnet, daß** der Gehalt an Teilchen, ausgedrückt in Calciumacetylacetonat oder Magnesiumacetylacetonat, zwischen 0,01 g und 5 g pro 100 g halogeniertes Polymer und ganz besonders zwischen 0,05 g und 2 g beträgt, bezogen auf die gleiche Referenz.

## Claims

1. Particles comprising calcium or magnesium acetylacetonate or a combination thereof, partially or completely coated with at least one compatibilizer selected from:
• saturated or unsaturated alcohols containing 12 to 30 carbon atoms;
• saturated or unsaturated, carboxylic or sulphonic acids containing 12 to 30 carbon atoms, which may or may not be substituted with at least one hydroxyl group, or derivatives thereof;
• phosphates or titanates comprising at least one saturated or unsaturated chain containing 12 to 30 carbon atoms;
• β-diketone compounds having at least one chain containing at least 7 carbon atoms;
• waxes;
• polyols;
• epoxidized vegetable oils;
• polysiloxane oils or resins, or silanes.

2. Particles according to the preceding claim, **characterized in that** the compatibilizer is an alcohol selected from saturated or unsaturated, aliphatic monoalcohols containing 12 to 30 carbon atoms.

3. Particles according to the preceding claim, **characterized in that** the alcohol is selected from lauric, myristic, stearic, isostearic, cetyl, behenic, lauroleic, oleic, erucic and linoleic alcohols.

4. Particles according to Claim 1, **characterized in that** the compatibilizer is selected from stearic, lauric, myristic, palmitic, oleic, ricinoleic and behenic (docosanoic) acids, linoleic acid, linolenic acid, ricinoleic acid and hydroxystearic acid, or any other acid originating from glycerides or triglycerides, whether natural or otherwise.

5. Particles according to Claim 1, **characterized in that** the compatibilizer is an acid derivative selected from esters of the aforementioned carboxylic acids, these being obtained from monoalcohols containing 1 to 30 carbon atoms, or monoesters or polyesters obtained from polyols.

6. Particles according to Claim 1, **characterized in that** the compatibilizer is a derivative selected from alkali metal, alkaline-earth metal, aluminium, lanthanum and zinc salts of the aforementioned carboxylic acids.

7. Particles according to Claim 1, **characterized in that** the compatibilizer is dodecylbenzenesulphonic acid.

8. Particles according to Claim 1, **characterized in that** the compatibilizer is a β-diketone compound of formula R¹COCHR²COR³, in which the radical R¹ represents a linear or branched, substituted or unsubstituted, C₇-C₃₀ hydrocarbon radical, the radical R³ represents a linear or branched, substituted or unsubstituted, C₁-C₃₀ hydrocarbon radical and the radical R² represents a hydrogen atom or a linear or branched, C₁-C₄ hydrocarbon radical.

9. Particles according to Claim 1, **characterized in that** the compatibilizer is selected from waxes such as montan waxes, polyethylene waxes or their oxidized derivatives, as well as paraffins.

10. Particles according to Claim 1, **characterized in that** the compatibilizer is selected from polyols containing 2 to 32 carbon atoms, having two to nine hydroxyl groups, it being a matter of indifference whether the hydroxyl groups are in neighbouring positions or not, or else polyvinyl alcohols, or polyols comprising isocyanurate groups.

11. Particles according to the preceding claim, **characterized in that** the polyol is selected from propylene glycol, butylene glycol, butanediol, pentanediol, hexanediol, dodecanediol, neopentyl glycol, polyols, such as trimethylol propane, pentaerythritol, dipentaerythritol, tripentaerythritol, xylitol, mannitol, sorbitol and glycerol, mixtures of glycerol oligomers having a degree of polymerization of 2 to 10, hydroxystearic alcohol and ricinoleic alcohol.

12. Particles according to Claim 1, **characterized in that** the compatibilizer is an epoxidized vegetable oil such as epoxidized soybean oil or epoxidized castor oil.

13. Particles according to Claim 1, **characterized in that** the compatibilizer is a polysiloxane oil corresponding to the following general formula: YO-[(R)Si(R)-O]ₓ-Y, in which formula R, which are identical or different, represent an alkyl radical containing 1 to 3 carbon atoms, and preferably a methyl, or a hydrogen atom provided that only one of the two radicals is a hydrogen, Y represents a hydrogen atom or (R')₃Si, with R', which are identical or different, representing an alkyl radical containing 1 to 3 carbon atoms, preferably methyl, with x varying between 5 and 300.

14. Particles according to the preceding claim, **characterized in that** the compatibilizer is a polysiloxane oil selected from functionalized polymethylsiloxane oils such as γ-hydroxypropylenated oils.

15. Particles according to Claim 1, **characterized in that** the compatibilizer is selected from polysiloxane resins obtained by the action of [lacuna] polysiloxane oils carrying vinyl groups, in the presence of a platinum-based catalyst, or else those obtained by the hydrolysis and self-condensation of at least one silane of formula (RO)₃SiF or (RO)₂(Me)SiF, in which R, which are identical or different, represent an alkyl radical containing 1 to 4 carbon atoms and F represents more particularly the following radicals:
**-CH=CH**_{**2**}**,**
**-(CH**_{**2**}**)**_{**3**}**OH, -(CH**_{**2**}**)**_{**3**}**-NH**_{**2**}**, -(CH**_{**2**}**)**_{**3**}**NHCH**_{**2**}**CH**_{**2**}**NH**_{**2**}**,** 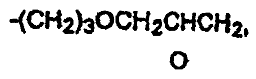
**-(CH**_{**2**}**)**_{**3**}**O-CO-CH=CH**_{**2**}**, -(CH**_{**2**}**)**_{**3**}**O-CO-(CH**_{**3**}**)CH=CH**_{**2**}**,**
or from the aforementioned silanes as such.

16. Particles according to any one of the preceding claims, **characterized in that** the proportion by weight of compatibilizer with respect to the calcium or magnesium acetylacetonate is between 0.1 and 20% by weight with respect to the weight of calcium or magnesium acetylacetonate, more particularly between 0.1 and 10% by weight with respect to the weight of calcium or magnesium acetylacetonate.

17. Process for preparing the particles according to any one of the preceding claims, **characterized in that** the aforementioned acetylacetonate is brought into contact with at least one compatibilizer, in the presence of a solvent and/or a dispersant.

18. Process according to the preceding claim, **characterized in that** the solvent and/or dispersant is selected from water, C₁-C₅ monoalcohols, such as especially methanol and ethanol, C₂-C₆ ethers, such as dimethyl ether, methyl ethyl ether and diethyl ether, and hydrocarbons such as, inter alia, hexane.

19. Use of particles according to any one of Claims 1 to 16 as an additive in formulations comprising at least one halogenated polymer.

20. Use according to the preceding claim, **characterized in that** the content of particles, expressed as calcium or magnesium acetylacetonate, is between 0.01 and 5 g per 100 g of halogenated polymer, more particularly between 0.05 and 2 g with respect to the same reference.
